# EUROPEAN PATENT APPLICATION

(11) **EP 4 574 163 A1**
(43) Date of publication of application: **25.06.2025**
(21) Application number: 23307386.5
(22) Date of filing: 22.12.2023
(51) Int. Cl.: A61K 38/16, A61P 3/10, C07K 14/00

(54) **ANTIHYPERGLYCEMIC COMPOUNDS AND METHODS**

(71) Applicant: Université Paris Cité, 75006 Paris (FR); Centre National de la Recherche Scientifique, 75016 Paris (FR)
(72) Inventor: MAGNAN, Christophe, 75013 PARIS (FR); DENOM, Jessica, 75013 PARIS (FR)
(74) Representative: Vial, Lionel

(57) **Abstract**

The present invention relate a peptide comprising, or consisting of:
- SEQ ID NO: 1, or
- a sequence having at least 80% identity with SEQ ID NO: 1,

or a microorganism comprising or secreting the peptide,
for use as an antihyperglycemic agent in an individual.

## Description

### Field of the invention

The present invention relates to antihyperglycemic compounds and methods.

### Technical background

Metformin has been used successfully for more than half a century in the treatment of type 2 diabetes. Due to its efficacy and limited side effects, metformin is recommended, in the absence of contraindications, as the first-line oral antidiabetic drug for treating patients with type 2 diabetes by the American diabetes *association* and the *European association of the study of diabetes*. Metformin reduces hyperglycemia without the risk of hypoglycemia, unlike other antidiabetic drugs such as sulfonylureas and insulin. For this reason, it is considered an antihyperglycemic agent. Metformin also improves insulin sensitivity, leading to a reduction in insulin resistance and a decrease in plasma concentrations of this hormone.

Animal and human studies have shown that metformin, a biguanide, acts in the liver, where it inhibits gluconeogenesis by blocking a mitochondrial redox shuttle. However, a full understanding of metformin's mechanism of action remains elusive.

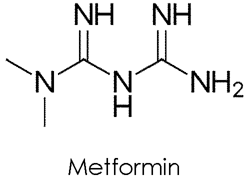

Although having a favorable safety profile, metformin is not deprived of side effects, as its causes adverse effects in as many as 25% of patients, particularly diarrhea and nausea (Flory & Lipska (2019) *JAMA* **321**:1926-1927). Besides, biguanides increase the risk of lactic acidosis. Lactic acidosis results from an increase in anaerobic glycolysis, which leads to a build-up of lactate in the circulation, causing a decrease in blood pH, which causes shock. Its incidence with metformin is estimated at 3 cases per 100,000 patients peryear. As such, metformin is contraindicated in all pathological situations that can cause either tissue hypoxia or ischemia, as in heart or lung failure, or an accumulation of metformin in the body due to lack of elimination, as in the case of renal or hepatic failure (Foretz & Viollet (2014) Med Sci (Paris) 30:82-92).

Accordingly, there is a need for alternative antihyperglycemic agents, in particular devoid of metformin's side effects.

### Summary of the invention

The present invention arises from the unexpected finding, by the inventors, that the MccJ25 peptide (SEQ ID NO: 1) had antihyperglycemic effects in glucose-intolerant obese mice.

As such, the present invention relates to a peptide comprising, or consisting of:
- SEQ ID NO: 1, or
- a sequence having at least 80% identity with SEQ ID NO: 1,

or a microorganism comprising or secreting the peptide,
for use as an antihyperglycemic agent in an individual.

In an embodiment of the invention, the peptide or microorganism is in combination with at least one other antihyperglycemic agent.

The present invention also relates to a pharmaceutical composition comprising:
- a peptide or microorganism as defined above, and
- at least one other antihyperglycemic agent,
as active ingredients, and optionally at least one pharmaceutically acceptable carrier or excipient.

In an embodiment of the invention, the pharmaceutical composition as defined above is for use as an antihyperglycemic agent in an individual.

The present invention also relates to products containing:
- a peptide or microorganism as defined above, and
- at least one other antihyperglycemic agent,
as a combined preparation for simultaneous, separate or sequential use as an antihyperglycemic agent in an individual.

The present invention also relates to the use of a peptide comprising, or consisting of:
- SEQ ID NO: 1, or
- a sequence having at least 80% identity with SEQ ID NO: 1,

or of a microorganism comprising or secreting the peptide,
for the manufacture of an antihyperglycemic medicament.

In an embodiment of the invention, the medicament further comprises at least one other antihyperglycemic agent.

The present invention also relates to a method for preventing or treating hyperglycemia in an individual, comprising administering to the individual an effective amount of a peptide comprising, or consisting of:
- SEQ ID NO: 1, or
- a sequence having at least 80% identity with SEQ ID NO: 1,
or of a microorganism comprising or secreting the peptide,

In an embodiment of the invention, the medicament further comprises administering at least one other antihyperglycemic agent to the individual.

### Description of the invention

As a preliminary remark, it should be noted that the term "consisting of" means "constituted by", i.e. when an object "consists of" an element or several elements, the object cannot include other elements than those mentioned. In contrast, the term "comprising" means "including", "containing" or "encompassing", i.e. when an object "comprises" an element or elements, other elements than those mentioned can also be included in the object. In other words, when an object "comprises" an element or elements, it consists of the element(s) and possibly of other elements than these.

Besides, as one of skilled in the art will well understand, the expressions "substance or composition for use in the prevention or treatment of a disease" is equivalent to the expression "substance or composition for use in a method for the prevention or treatment of a disease".

### Peptide

As used herein, the terms "peptide" and "polypeptide" are used in their broadest sense to refer to a molecule of two or more amino acid residues. The amino acid residues may be linked by peptide bonds, or alternatively by other bonds, e.g. ester, ether, etc. However, the amino acid residues are preferably linked together by peptide bonds.

As used herein, the terms "amino acid" and "amino acid residue" encompass natural and non-natural or synthetic amino acids, including D- and L-forms, and amino acid analogues. An "amino acid analogue" is to be understood as a non-naturally occurring amino acid that differs from a corresponding naturally occurring amino acid in one or more atoms. For example, an amino acid analogue of cysteine may be homocysteine. However, the amino acids or amino acid residues are preferably naturally occurring.

As understood here, the percentage of identity between two peptide sequences can be determined by performing an optimal alignment along the entire length of the sequences, determining the number of aligned positions for which the amino acids are identical in each sequence and dividing this number by the total number of amino acids in the longer of the two sequences. The optimal alignment is the one that gives the highest percentage of identity between the two sequences.

Preferably, the peptide according to the invention comprises at most 100, 90, 80, 70, 60, 50, 40, 30, 25, 24, 23, 22, 21 or 20 amino acids. Preferably, the peptide according to the invention comprises at least 5, 6, 7, 8, 9, 10, 12, 15, 18, 20, 40, 50, 60, 70, 80 or 90 amino acids. Preferably, the peptide according to the invention comprises from 10 to 40 amino acids, more preferably from 15 to 35 amino acids.

The peptide according to the invention may comprise post-translational modifications, such as glycosylations, methylations, acylations, in particular by fatty acids or by an acetyl group, amidations, or phosphorylations. For example, the N-terminus of the peptide according to the invention may be acetylated or its C-terminus may be modified by amidation.

The sequence having at least 80% identity with SEQ ID NO: 1 according to the invention will preferably have at least 85%, 90%, 95%, or 98% identity with SEQ ID NO: 1.

Preferably, the peptide according to the invention consists of SEQ ID NO: 1. SEQ ID NO: 1 has the sequence GGAGHVPEYFVGIGTPISFYG.

Preferably, the peptide according to the invention is in a lasso configuration.

More preferably, the peptide according to the invention is the MccJ25 peptide, notably represented by the following formula (I):

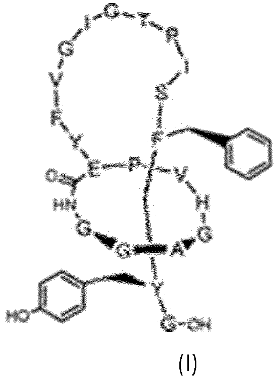

The polypeptide according to the invention can be prepared by any method known in the state of the art and in particular by extraction from bacteria naturally producing MccJ25 such as *Escherichia coli* (Wilson et al. (2003) J. Am. Chem. Soc. 125:12475-12483; Salomon & Farias (1992) J. Bacteriol. 174:7428-7435). It is also possible to prepare it by the recombinant route in prokaryotic cells (Ducasse et al. (2012) Chembiochem 13:371-380).

### Microorganism

As intended herein, a microorganism which comprises or secretes the peptide according to the invention, is a microorganism which produces or expresses the peptide according to the invention. The microorganism may express the peptide according to the invention naturally. The microorganism according to the invention may also have been engineered to express the peptide, in particular by recombinant methods.

The microorganism can be of any type suitable to be administered to the individual. Preferably, the microorganism is a bacterium or a yeast. More preferably, the microorganism is a probiotic bacterium or yeast. Even more preferably, the microorganism is selected from the group consisting of bacteria of a genus selected from the group consisting of:
- *Lactobacillus,* such as *L*. *reuteri, L*. *fermentum, L*. *johnsonii, L*. *brevis, L*. *delbruckii subsp. bulgaricus, L. casei, L. plantarum, L*. *acidophilus,* and *L*. *rhamnosus*;
- *Bifidobacterium,* such as *B. breve, B. longum, B*. *bifidum, B*. *animalus subsp. Lactis*, and *B. infantis*;
- *Lactococcus,* such as *L. lactis subsp lactis*;
- *Enterococcus,* such as *E. durans* and *E. faecium*;
- *Streptococcus,* such as *S. thermophilus*;
- *Pediococcus,* such as *P. acidilactici*;
- *Leuconostoc,* such as *L.* mesenteroides;
- *Bacillus,* such as *B. coagulans, B. subtilis* and *B. cereus;*
- *Escherichia,* such as *E. coli.*

### Individual

The individual or the subject according to the invention is an animal, preferably a mammal, and more preferably a human, in particular a man, a woman or a child.

It is preferred that the individual has an excess body fat, in particular in relation to his stature. As intended herein, the body fat or adipose mass or fat mass represents the mass of all the white adipose tissues of the body of the individual or the subject. Excess body fat is characterized with respect to normality and can be estimated by many methods well known to one of skilled in the art, including nuclear magnetic resonance (NMR), by calculating the body mass index (BMI) of the individual (body weight in kg relative to the square of the height in meters) or by measuring the waist-to-hip ratio or the abdominal perimeter of the individual. The body mass or weight of the individual or the subject according to the invention can be determined using a weighing scale.

It is also preferred that the individual according to the invention is either overweight or obese. According to a usual definition a human individual is considered overweight if his BMI is higher than or equal to 25 kg/m² and less than 30 kg/m² and the individual will be said obese if his BMI is higher than or equal to 30 kg/m². The individual according to the invention may notably present with severe obesity, in particular characterized in human by a BMI higher than or equal to 35 kg/m².

More generally, it is preferred that the individual according to the invention is a human and has a BMI higher than or equal to 25 kg/m², 26 kg/m², 27 kg/m², 28 kg/m², 29 kg/m², 30 kg/m², 31 kg/m², 32 kg/m², 33 kg/m², 34 kg/m², 35 kg/m² or 40 kg/m².

Besides, the individual according to the invention may also have an abdominal obesity, corresponding in particular to a visceral adipose tissue excess. According to a usual definition a male human individual has an abdominal obesity if the abdominal perimeter is higher than or equal to 94 cm, in particular higher than 102 cm and a female human individual has an abdominal obesity if the abdominal perimeter is higher than or equal to 80 cm, in particular higher than 88 cm. The abdominal perimeter measure is well known to one of skill in the art: abdomen circumference is thus preferably measured midway between the last floating rib and the top of the iliac crest in a standing individual in gentle expiration. According to another definition, a male individual has an abdominal obesity if the waist-to-hip ratio is above 0.9 and a female individual has an abdominal obesity if the waist-to-hip ration is above 0.85. The waist-to-hip ratio measurement is well known to one of skill in the art and is notably defined by the World Health Organization (WHO): waist circumference should be measured at the midpoint between the lower margin of the last palpable ribs and the top of the iliac crest, using a stretch-resistant tape that provides constant 100 g tension and hip circumference should be measured around the widest portion of the buttocks, with the tape parallel to the floor.

It is particularly preferred that the individual according to the invention is a man and presents with an abdominal perimeter higher than or equal to 90 cm, 91 cm, 92 cm, 93 cm, 94 cm, 95 cm, 96 cm, 97 cm, 98 cm, 99 cm, 100 cm, 101 cm or 102 cm. It is also preferred that the individual according to the invention is a woman and presents with an abdominal perimeter higher than or equal to 75 cm, 76 cm, 77 cm, 78 cm, 79 cm, 80 cm, 81 cm, 82 cm, 83 cm, 84 cm, 85 cm, 86 cm, 87 cm or 88 cm. Alternatively, it is particularly preferred that the individual according to the invention is a man and presents with a waist-to-hip ratio higher than or equal to 0.8, 0.85, 0.9, 0.95, 1, 1.05 or 1.1. It is also preferred that the individual according to the invention is a woman and presents with a waist-to-hip ration higher than or equal to 0.7, 0.75, 0.8, 0.85, 0.9, 0.95 or 1.

It is further preferred that the individual according to the invention has, suffers from, presents, or has been diagnosed with at least one of hyperglycemia, in particular chronic hyperglycemia, glucose intolerance, prediabetes, insulin resistance, and type I or type II diabetes, metabolic syndrome, more preferably the individual according to the invention has insulin resistance.

As intended herein, hyperglycemia relates to a blood or plasma glucose concentration, i.e. a glycemia, higher than 6.1 mmol/L or 1.10 g/L, in a fasting individual. When the blood or plasma glucose level is higher than 7 mmol/L or 1,26 g/L in a fasting individual, the individual is considered to have diabetes. Individuals with prediabetes have a blood or plasma glucose concentration higher than 6.1 mmol/L but lower than 7 mmol/L.

### Treatment

As intended herein, an antihyperglycemic agent is capable of lowering or reducing glycemia in an individual having hyperglycemia, in particular chronic hyperglycemia. Preferably, an antihyperglycemic agent according to the invention is capable of restoring a normal level of glycemia, *i.e.* a glycemia lower than or equal to 6.1 mmol/L or 1.10 g/L, in an individual having hyperglycemia, in particular chronic hyperglycemia. Preferably also, an antihyperglycemic agent is does not reduce or lower a normal glycemia.

Preferably, the peptide or microorganism, the pharmaceutical composition or the products according to the invention are for use in a method for prevention or treatment of a condition or disease selected from the group consisting of prediabetes, insulin resistance, and type I or type II diabetes.

Preferably, the peptide or microorganism, the pharmaceutical composition or the products according to the invention are for use in a method for prevention or treatment of metabolic syndrome.

Advantageously, the peptide or microorganism, the pharmaceutical composition or the products according to the invention reduces the gut Firmicutes/Bacteroidota ratio in treated individuals.

### Administration

As intended herein, "combined" or "in combination" means that the peptide or microorganism as defined above, is administered at the same time than another antihyperglycemic agent, either together, *i.e.* at the same administration site, or separately, or at different times, provided that the time period during which the peptide or microorganism as defined above, exerts its effects on the individual and the time period during which the other hyperglycemic agent exerts its pharmacological effects on the individual, at least partially intersect.

Preferably, the peptide or microorganism, the pharmaceutical composition or the products according to the invention are administered to the individual by the oral, mucosal, intramuscular, subcutaneous, intravenous or intradermal route.

The peptide or microorganism or the other antihyperglycemic agent may associated to or combined with at least one pharmaceutically acceptable carrier or excipient.

As intended herein "pharmaceutically acceptable carrier or excipient "refers to any material suitable to be associated to or combined with an active ingredient and to be administered to an individual. Preferably, the pharmaceutically acceptable carrier or excipient according to the invention, includes but is not limited to any of the standard carriers or excipients known to one of skilled in the art such as water, glycerin, alcohol, oil emulsion, water emulsion, buffered saline solution, preservative, stabilizer and wetting agents.

In particular the pharmaceutically acceptable carrier or excipient according to the invention is a gastro-resistant carrier, excipient or coating, i.e. a carrier, excipient or coating that prevents the degradation of the peptide or the microorganism according to the invention when passing through the stomach after an oral administration. Such gastro-resistant carriers, excipients or coating (*i.e*. an enteric coating) are well known to a person skilled in the art and notably selected from the list consisting of:
- Methyl acrylate-methacrylic acid copolymers;
- Cellulose acetate phthalate (CAP);
- Cellulose acetate succinate;
- Hydroxypropyl methyl cellulose phthalate;
- Hydroxypropyl methyl cellulose acetate succinate (hypromellose acetate succinate);
- Polyvinyl acetate phthalate (PVAP);
- Methyl methacrylate-methacrylic acid copolymers;
- Shellac;
- Cellulose acetate trimellitate;
- Sodium alginate;
- Zein, and
- Chitosan.

### Other antihyperglycemic agent

Preferably, the other antihyperglycemic agent is selected from the group consisting of:
- Sulfonylureas, such as glipizide, glyburide, gliclazide, and glimepiride;
- Meglitinides, such as repaglinide and nateglinide;
- Biguanides, such as metformin;
- Thiazolidinediones, such as rosiglitazone and pioglitazone;
- α-Glucosidase inhibitors, such as acarbose, miglitol, and voglibose;
- DPP-4 inhibitors , such as sitagliptin, saxagliptin, vildagliptin, linagliptin, and alogliptin;
- SGLT2 inhibitors, such as dapagliflozin and canagliflozin, and
- Cycloset, such as bromocriptine.

More preferably, the other antihyperglycemic agent is metformin.

The invention will be further described by the following non-limiting figures and examples.

### Description of the figures

Figure 1

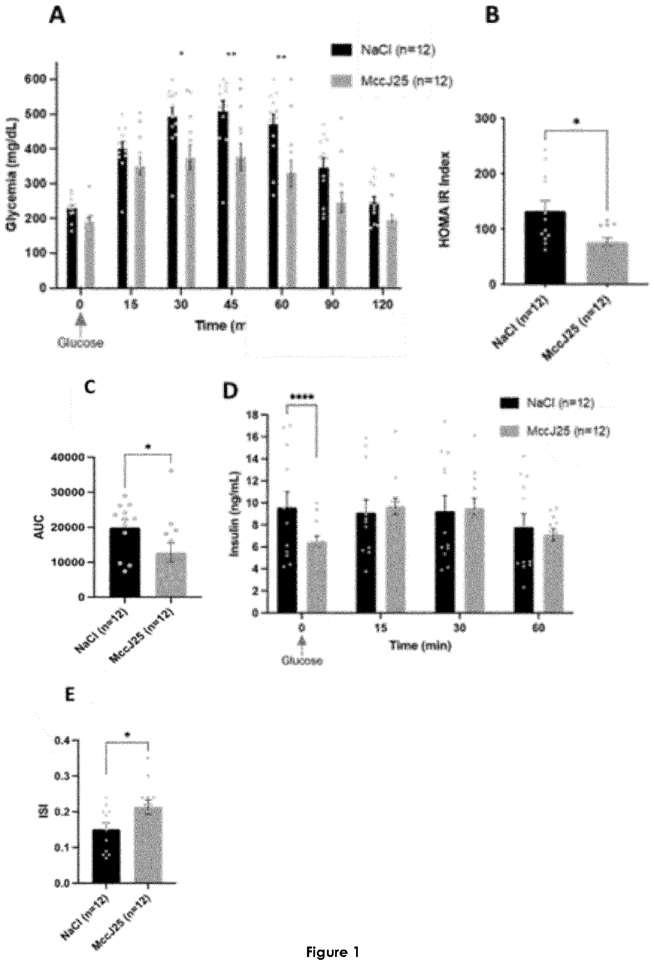

   Figure 1 represents the *in vivo* effect of intraperitoneally administration of MccJ25 (daily injection, 20 mg/kg during 5 days). Figure 1A: basal (t0) and time course of glycemia following oral ingestion of glucose (3 g/kg); Figure 1B: HOMA-IR; Figure 1C: Area under the curve (AUC) of glycemia in response to oral glucose ingestion; Figure 1D: basal (t0) and time course of plasma insulin following oral ingestion of glucose; Figure 1E: Insulin sensitivity index. *p<0.05, **p<0.01, ****p<0.0001 vs NaCl 0.9% (controls).
Figure 2

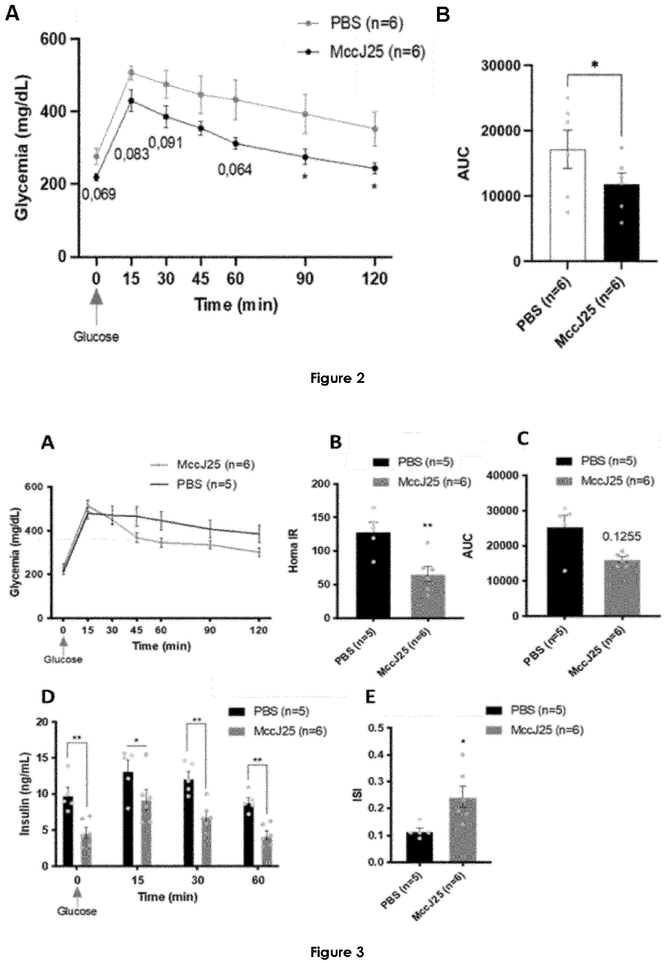

   Figure 2 represents the time course of glycemia (Figure 2A) and area under the course (Figure 2B) of glycemia following glucose gavage (3g/kg) in mice treated subcutaneously with PBS (placebo) or MccJ25 (20 mg/kg) for 5 days. *p<0.05 vs placebo.
Figure 3
   Figure 3 represents the *in vivo* effect of oral administration of MccJ25 (daily injection, 20 mg/kg for 5 days). Figure 3A: basal (t0) and time course of glycemia following oral ingestion of glucose (3 g/kg); Figure 3B: HOMA-IR; Figure 3C: Area under the curve (AUC) of glycemia in response to oral glucose ingestion; Figure 3D: basal (t0) and time course of plasma insulin following oral ingestion of glucose; Figure 3E: Insulin sensitivity index. *p<0.05, **p<0.01 vs PBS (controls).

### EXAMPLES

### Example 1 - Intraperitoneal administration of MccJ25 or placebo in HFD mice

12 weeks old C57B6 mice were fed a high-fat diet (HFD, 40% fat) for 2 months to obtain obese glucose-resistant mice as a model of pre-diabetes (Cruciani-Guglielmacci et al. (2017) Mol. Metab. 6:340-351).
Mice were then treated for 5 days (daily intraperitoneal injection) either with MccJ25 (20 mg/kg) (n = 12) or NaCl 0.9 % (placebo) (n = 12).

In the basal state (t0, Figure 1A), intraperitoneally administration of MccJ25 tended to decrease basal glycemia and significantly reduced basal insulinemia (Figure 1D) compared to controls. This reflected by a significant decrease in insulin resistance as evidenced by the Homeostatic Model Assessment for Insulin Resistance (HOMA-IR) index (Figure 1B). During oral glucose tolerance test (OGTT, 3g/kg), hyperglycemia was less marked (and significantly lower at times 30, 45 and 60 min compared to placebo) with equal insulin levels (Figure 1A, Figure 1D), suggesting an increase in insulin sensitivity as highlighted by the increased insulin sensitivity index ISI in treated mice vs placebo (Figure 1E). Finally, area under the curve (AUC) of glycemia following oral glucose administration (3g/kg ) was significantly lower in MccJ25 treated mice compared to placebo (Figure 1C).

### Example 2 - Subcutaneous administration of MccJ25 or placebo in HFD mice

Example 1 was reproduced with daily subcutaneous (SC) injections for 5 days of MccJ25 (20 mg/kg) (n = 6) vs PBS (placebo) (n = 6).

The results are shown in Figure 2. As with intraperitoneal injection, subcutaneous administration of MccJ25 improved glucose tolerance (Figure 2A) as evidenced by a significant decrease in the area under the curve (AUC) for glycemia (Figure 2B).

### Example 3 - Oral administration of MccJ25 or placebo in HFD mice

Example 1 was reproduced with daily oral administrations for 5 days of MccJ25 (20 mg/kg) (n = 6) vs PBS (placebo) (n = 5).

MccJ25 significantly decreased basal (time 0) insulinemia (Figure 3D). Insulin resistance (HOMA-IR) was significantly decreased (Figure 3B). During OGTT, MccJ25 lead to a decrease in glucose tolerance (3A) and AUC (3C). A significant decrease in insulin following glucose administration (at times 15, 30 and 60 min, Figure 3D) was evidenced because of increased insulin sensitivity (Figure 3E).

A shotgun metagenomics and analysis of the microbiome showed differences in gene patterns in treated versus placebo. Among the most impacted genes were TonB and genes of the ABC transporter family. Most genes belong to the phylum Firmicutes (and *Lachnospiracae* species) and Bacteroidota (and *Bacteroides* species). A decrease in the Firmicutes/Bacteroidota ratio in obese mice treated with MccJ25 compared to placebo was evidenced that could be related to improvement of the observed glucose homeostasis.

## Claims

1. A peptide comprising, or consisting of:
- SEQ ID NO: 1, or
- a sequence having at least 80% identity with SEQ ID NO: 1,
or a microorganism comprising or secreting the peptide,
for use as an antihyperglycemic agent in an individual.

2. The peptide or microorganism for use according to claim 1, wherein the peptide consists of SEQ ID NO: 1.

3. The peptide or microorganism for use according to claim 1 or 2, wherein the peptide is in a lasso configuration.

4. The peptide or microorganism for use according to anyone of claims 1 to 3, wherein the individual has insulin resistance.

5. The peptide or microorganism for use according to anyone of claims 1 to 4, in a method for prevention or treatment of a condition or disease selected from the group consisting of prediabetes, insulin resistance, and type I or type II diabetes.

6. The peptide or microorganism for use according to anyone of claims 1 to 5, in a method for prevention or treatment of metabolic syndrome.

7. The peptide or microorganism for use according to anyone of claims 1 to 6, wherein the peptide or microorganism is administered to the individual by the oral, mucosal, intramuscular, subcutaneous, intravenous or intradermal route.

8. The peptide or microorganism for use according to anyone of claims 1 to 7, wherein the peptide or microorganism is in combination with at least one other antihyperglycemic agent.

9. The peptide for use according to claim 8, wherein the other antihyperglycemic agent is metformin.

10. A pharmaceutical composition comprising:
- a peptide or microorganism as defined in anyone of claims 1 to 3, and
- at least one other antihyperglycemic agent,
as active ingredients, and optionally at least one pharmaceutically acceptable carrier or excipient.

11. The pharmaceutical composition according to claim 10, wherein the other antihyperglycemic agent is metformin.

12. The pharmaceutical composition according to claim 10 or 11, wherein the composition is suitable to be administered by the oral, mucosal, intramuscular, subcutaneous, intravenous or intradermal route.

13. The pharmaceutical composition according to anyone of claims 10 to 12, for use as an antihyperglycemic agent in an individual.

14. Products containing:
- a peptide or microorganism as defined in anyone of claims 1 to 3, and
- at least one other antihyperglycemic agent,
as a combined preparation for simultaneous, separate or sequential use as an antihyperglycemic agent in an individual.

15. The products according to claim 14, wherein the other antihyperglycemic agent is metformin.
